# EUROPÄISCHE PATENTSCHRIFT

(11) **EP 1 061 128 B1**
(45) Veröffentlichungstag und Bekanntmachung des Hinweises auf die Patenterteilung: **13.10.2004**
(21) Anmeldenummer: 99111762.3
(22) Anmeldetag: 18.06.1999
(51) Int. Cl.: C12M 1/40, C12P 21/02, B01J 19/24, B01J 19/00

(54) **Verfahren und Vorrichtung zur Durchführung biochemischer Reaktionen mit hohem Durchsatz**
Method and device for carrying out high throughput biochemical reactions
Procédé et dispositif pour la mise en oeuvre de réactions biochimiques à haut débit

(43) Veröffentlichungstag der Anmeldung: 20.12.2000
(73) Patentinhaber: Roche Diagnostics GmbH, 68298 Mannheim (DE)
(72) Erfinder: Metzler, Thomas, 80337 München (DE); Schels, Hans, 80687 München (DE)

(56) Entgegenhaltungen:
- EP-A- 0 894 852
- WO-A-97/04074
- DE-A- 3 723 004
- DE-A- 4 237 113
- US-A- 4 642 220
- US-A- 5 362 624
- US-A- 5 478 730
- KIM D M ET AL: "A semicontinuous prokaryotic coupled transcription/translation system using a dialysis membrane." BIOTECHNOLOGY PROGRESS., Bd. 12, Nr. 5, September 1996 (1996-09), Seiten 645-649, XP002106860 ISSN: 8756-7938
- PATENT ABSTRACTS OF JAPAN vol. 1998, no. 04, 31. März 1998 (1998-03-31) & JP 09 322755 A (RIKAGAKU KENKYUSHO & SAINIKUSU K.K.), 16. Dezember 1997 (1997-12-16) & DATABASE WPI Section Ch, Week 199810 Derwent Publications Ltd., London, GB; Class B04, AN 1998-103992 & JP 09 322755 A (RIKAGAKU KENKYUSHO & SAINIKUSU K.K.), 16. Dezember 1997 (1997-12-16)

## Beschreibung

Die Erfindung betrifft ein Verfahren sowie eine Vorrichtung zur Durchführung biochemischer Reaktionen, insbesondere für die Polypeptid-Biosynthesebzw. zur gekoppelten in vitro-Transkription und Translation von Proteinen in einem zellfreien System mit Hilfe einer "Multichannel"-Dialyse-Vorrichtung, wodurch die Synthese unterschiedlicher Proteine nebeneinander in ausreichenden Ausbeuten unter einfachen und reproduzierbaren Bedingungen möglich ist.

Das Prinzip der zellfreien in vitro-Proteinbiosynthese unter Einsatz von Dialysemembranen ist seit mehreren Jahren bekannt und besteht im wesentlichen darin, daß zwei getrennte Kammern (eine für die Reaktionsmischung, eine für die Versorgungslösung) über eine Membran mit geeigneter Porengröße in Verbindung stehen. In der Reaktionskammer erfolgt die Proteinsynthese. In der Versorgungskammer ist eine Lösung mit sämtlichen für die Transkription bzw. Translation benötigten Reaktionskomponenten, die während der laufenden Proteinbiosynthese in der Reaktionsmischung verbraucht werden, enthalten. Bedingt durch die Tatsache, daß beide Kammern über eine semipermeablen Membran in Verbindung stehen, können verbrauchte Reaktionskomponenten in der Reaktionskammer, gegebenenfalls mittels einer entsprechenden Pumpeinrichtung, fortlaufend durch neue Komponenten aus der Versorgungskammer ersetzt werden, wodurch die Synthese über einen deutlich längeren Zeitraum im Vergleich zum statischen System, d.h. einer entsprechenden, in einem einzelnen Reaktionsgefäß stattfindenden Reaktion, aufrechterhalten bleibt. Entsprechende Verfahren für die zellfreie in vitro-Biosynthese nach dem "continous-flow" bzw. "continous exchange"-Prinzip sind beispielsweise in US 5.478.730, EP 0 593 757 und von Spirin et al. in Science vol. 242, 1988, S. 1162-1164 beschrieben.

Das gemäß der Patentschrift US 5.478.730 verwendete Translationssystem enthält beispielsweise eine Quelle von mRNA, die das Polypeptid codiert. Weiter sind in dem zellfreien Synthesesystem Ribosomen, tRNA, Aminosäuren, ATP und GTP enthalten. Die Translation der mRNA mit Hilfe der tRNA führt zur Produktion des jeweiligen Polypeptids, wobei zugleich Nebenprodukte und Abfallstoffe mit niedrigerem Molekulargewicht entstehen. Diese können durch eine semipermeable Membran, die den Raum, in dem das Synthesesystem enthalten ist, von einem Versorgungsraum trennt, in den Versorgungsraum übertreten. Der Versorgungsraum enthält eine als Versorgungsmedium wirkende Flüssigkeit, in der ATP, GTP und Aminosäuren enthalten sind. Diese Komponenten werden durch die semipermeable Membran dem Synthesesystem zugeführt, um den Verbrauch während der Biosynthesereaktion zu ersetzen. Der Durchtritt durch die semipermeable Membran ist möglich, weil ihr Molekulargewicht unterhalb von deren Durchlaßgrenze liegt. Zugleich gelangen Produkte der biochemischen Reaktion und andere Substanzen, deren Molekulargewicht unterhalb der Durchlaßgrenze der Barriere liegt, aus dem Reaktionsraum in den Versorgungsraum. Die semipermeable Membran ist gemäß der US Patentschrift 5.478.730 beispielsweise eine Ultrafiltrationsmembran in Form von Membran-Hohlfasern.

Die US Patentschrift 5.478.730 enthält umfangreiche weitere Erläuterungen über geeignete Zusammensetzungen des Synthesesystems und der Versorgungsflüssigkeit. Insoweit bezieht sich die vorliegende Erfindung auf den vorbekannten Stand der Technik, wie er insbesondere dieser US Patentschrift und den darin zitierten Literaturstellen zu entnehmen ist.

Das europäische Patent 0 593 757 beschreibt die Anwendung der gekoppelten Transkription und Translation tür entsprechende in vitro-Biosyntheseverfahren, wobei im wesentlichen zusätzlich eine RNA-Polymerase der Reaktionsmischung zugesetzt wurde.

Darüber hinaus sind diverse Dialyse-Vorrichtungen bzw. -Membranen bekannt. Man unterscheidet dabei im wesentlichen sogenannte "single tube" und "Multi-channel"-Ausführungen. Die beschriebenen bzw. kommerziell erhältlichen und für die in vitro-Proteinbiosynthese geeigneten Dialysematerialien beruhen ausschließlich auf dem "single tube"-Prinzip (z.B. EP 99 91 01 418.4; Promega Notes 1997; D.-M. Kim und C.-Y. Choi, Biotechnol. Prog. vol. 12, 1996, S. 645-649). Ein hoher Durchsatz bzw. die Synthese unterschiedlicher Proteine nebeneinander, darüber hinaus ohne aufwendige Aufreinigungsmaßnahmen, ist jedoch mittels dieser Dialyse-Hilfsmittel nicht möglich. Ferner sind die Dialyse-Vorrichtungen nach dem "single-tube"-Prinzip mit dem Nachteil verbunden, daß sie technisch und zeitlich aufwendig zu handhaben sind.

Ferner sind Mikrotiterplatten mit Vertiefungen, welche am unteren Ende mit einer porösen Membran versehen sind, beschrieben bzw. werden kommerziell angeboten. Sogenannte "Multichannel"-Ausführungen werden ausschließlich für Zellkulturanwendungen bzw. für Filtrationsoder Umpufferungsvorgänge eingesetzt (z.B. Ultrafiltrationsmembranen von Milipore; Slide-A-Lyzer® MINI Dialysis Unit von Pierce; WO 97/04074). Ein Austausch zwischen Flüssigkeiten unterschiedlicher Konzentrationen, wie sie bei der in vitro-Transkription bzw. in vitro-Translation verwendet werden, ist mit diesen "Multi-channel"-Vorrichtungen jedoch nicht möglich, weshalb diese für in vitro-Proteinsynthese-Anwendungen, beispielsweise mit kontinuierlicher Zufuhr bzw. Entnahme von Komponenten nicht geeignet sind.

Der Erfindung liegt somit die Aufgabe zugrunde ein Verfahren und eine Vorrichtung zur Verfügung zu stellen, mit denen die parallele Durchführung der zellfreien Polypeptid-Biosynthese mit hoher Effektivität und zugleich einfach und gut reproduzierbar möglich ist.

Die Aufgabe wird gelöst durch eine Vorrichtung zur Durchführung zellfreier Polypeptid-Biosynthesen, bestehend aus einem äußeren Gehäuse, das ein inneres Gehäuse mit eingearbeiteten Vertiefungen und eine Versorgungskammer umschließt, wobei die Vertiefungen des inneren Gehäuses während der biochemischen Reaktion jeweils ein produzierendes System enthalten, die Versorgungsflüssigkeit der biochemischen Reaktion eine Versorgungsflüssigkeit enthält und die Vertiefungen des inneren Gehäuses und die Versorgungskammer durch eine semipermeable Membran getrennt sind, dadurch gekennzeichnet, daß das innere Gehäuse mindestens zwei Vertiefungen aufweist, deren untere Enden mit der semipermeablen Membran abgeschlossen sind und deren obere Enden aus der in der Versorgungskammer enthaltenen Versorgungsflüssigkeit herausragen, und mit Mitteln zur Bewegung und Temperierung der produzierenden Systeme sowie der Versorgungsflüssigkeit verbunden ist, wobei die Mittel zur Bewegung dergestalt sind, dass die Durchmischung des produzierenden Systems und der Versorgungslösung simultan erfolgt.

Als äußere Gehäuse kommen erfindungsgemäß prinzipiell schalen- oder zylinderförmige Gefäße in Betracht, die zur Flüssigkeitsaufnahme befähigt sind und in denen ein geometrisch kleineres, inneres Gehäuse einsetzbar und gegebenenfalls fixierbar ist. Das innere Gehäuse kann, entsprechend dem äußeren Gehäuse, größen- und formgemäß beliebige äußere Ausmaße haben. Beispielsweise sind runde, rechteckige oder quadratische Anordnungen für das äußere Gehäuse mit Volumina von ca. 10 ml bis mehrere Liter geeignet. Vorteilhaft ist, wenn das innere Gehäuse möglichst weitgehend an die Geometrie des äußeren Gehäuses angepaßt ist.

Das innere Gehäuse weist eine oder mehrere Vertiefungen auf, bevorzugt ist eine Anzahl zwei oder mehr, z. B. sechs, acht, 24, 64, 96, 384 etc. Es sind jedoch prinzipiell auch innere Gehäuse, beispielsweise in Form von Mikrotiterplatten, die mehrere hundert bis 1000 oder mehr Vertiefungen für die erfindungsgemäße Vorrichtung aufweisen, einsetzbar. Die Vertiefungen sind in der Regel aus einem inertem Material, wie beispielsweise Polyethylen, gefertigt und können für Volumina von ca. 50 µl bis zu mehreren Millilitern, d.h. in der Größenordnung von 10 ml ausgerichtet sein. Vorteilhaft sind konisch zulaufende Vertiefungen, die am unteren Ende mit einer semipermeablen Membran, wie z. B. einer Dialysemembran mit einer Porengröße von 3 bis 100 Kilo Dalton, verschlossen sind. Prinzipiell können erfindungsgemäß sämtliche gängigen Dialyse- bzw. Ultrafiltrationsmembranen mit entsprechender Porengröße eingesetzt werden. Besonders geeignet haben sich Dialysemembranen mit einer Porengröße zwischen ca. 10 und 14 Kilo Dalton erwiesen. Dadurch können insbesondere störende niedermolekulare, inhibitorische Substanzen, die bei der in vitro-Biosynthese entstehen, abgetrennt werden.

Darüber hinaus hat sich als vorteilhaft erwiesen, wenn die Wände der einzelnen Vertiefungen ("Wells") des inneren Gehäuses mit die in vitro synthetisierten Proteine bzw. Peptide spezifisch bindenden Komponenten beschichtet sind. Entsprechende Komponenten sind insbesondere solche, die zur Reinigung von tag-enthaltenden Proteinen geeignet sind. Das in den Wells in vitro synthetisierte Protein (tag enthaltend) bindet beispielsweise an die Mikrotiterplatte und kann anschließend direkt, gegebenenfalls nach Waschen mit geeigneten Puffern, gereinigt werden bzw. durch geeignete Reagenzien in sauberer Form eluiert werden. Beispielhaft soll hier als sogenanntes Protein-tag Strep-Tag II (8AS-Sequenz, s. DE 42 37 113), das entweder N- oder C-terminal an das in vitro-synthetisierte Protein gebunden sein kann, angeführt werden. Als Beschichtungssubstanzen können beispielsweise Streptactin, Streptavidin oder Avidin eingesetzt werden. Verfahren zur Beschichtung entsprechender Oberflächen sind dem Fachmann bekannt. Alternativ ist auch möglich, die synthetisierten, tag-tragenden Proteine mittels entsprechend beschichteter Glas- bzw. Magnetpartikel aus der Reaktionsmischung der einzelnen Vertiefungen zu separieren.

Ferner ist die erfindungsgemäße Vorrichtung mit einer Rühr- oder Schüttelvorrichtung ausgestattet, um eine ausreichende Bewegung bzw. Diffusion der Reaktionslösung(en) und der Versorgungslösung zu garantieren. Als vorteilhaft hat sich erwiesen, wenn in der Versorgungskammer und gegebenenfalls in jeder einzelnen Vertiefung, in der eine Reaktion abläuft, ein Rührelement in Form eines Magnetrührers eingebracht ist. Zur Gewährleistung einer konstanten Temperatur während der biochemischen Reaktion - welche in der Regel zwischen 20° und 37°C beträgt - wird die gesamte Vorrichtung am einfachsten vollständig in einen verschließbaren Inkubator eingebracht oder unter einer temperierbaren Inkubationshaube gehalten. Erfindungsgemäß sind darüber hinaus kombinierte Schüttel- bzw. Rühr- und Thermostatisiergerät einsetzbar. Die Durchmischung der Reaktionsmischung(en), als auch der Versorgungslösung kann somit simultan mit Schüttel- bzw. Rührbewegungen geeigneter Frequenz sowie über einen längeren Zeitraum bei konstanter Temperatur erfolgen.

In der Regel ist ausreichend, die Reaktionsmischung(en) über einen Zeitraum von ca. 20 Stunden zu inkubieren, um das bzw. die gewünschten Proteine bzw. Peptide in ausreichenden Ausbeuten zu erhalten. Abhängig von dem zu synthetisierenden Protein bzw. der Optimierung der einzelnen Verfahrensparameter bzw. der genauen Zusammensetzung der Versorgungslösung können bereits nach ca. 6 Stunden ca. 25 bis 50 µg Protein /250 µl Reaktionslösung in einer Vertiefung (Well), was einer Konzentration von 100 bis 200 µg/ml entspricht, erzielt werden. Durch verlängerte Inkubationszeiten können darüber hinaus entsprechend bessere Ausbeuten erreicht werden, beispielsweise wurden für GFP Konzentrationen bis zu 500 µg/ml erhalten.

Eine weitere bevorzugte Ausführungsform der Erfindung ist, wenn abhängig von der Anzahl bzw. dem Gesamtvolumen der Vertiefungen des inneren Gehäuses das Volumen der Versorgungslösung bestimmt wird. Als Richtlinie gilt erfindungsgemäß, daß das Volumen der Versorgungslösung gleich der Summe aus der Anzahl der Vertiefungen und dem Volumen pro Vertiefung multipliziert mit dem Faktor 10 entspricht.

Die Zusammensetzung der Versorgungslösung entspricht im wesentlichen entsprechenden Lösungen für die zellfreie in vitro-Biosynthese des Standes der Technik. Dem Fachmann ist darüber hinaus geläufig, daß Versorgungslösungen für die zellfreie Proteinsynthese bestimmten üblichen Optimierungsmaßnahmen unterliegen, insbesondere je nach Art und Beschaffenheit der verwendeten Ribosomenfraktion, d.h. ob beispielsweise ein eukaryontisches oder prokaryontisches System als Grundlage für die zellfreie in vitro-Biosynthese verwendet wird. Als vorteilhaft hat sich darüber hinaus erwiesen, wenn die Versorgungslösung ein reduzierendes Mittel sowie - im Falle eines Lysats auf E. coli-Basis - einen entsprechenden Polymerase-Inhibitor und gegebenenfalls eine oder mehrere geeignete bakterizide Substanzen enthält.

Ein weiterer Gegenstand der Erfindung ist ein Verfahren zur Durchführung einer und insbesondere mehrerer parallel laufender biochemischer Reaktionen mittels der erfindungsgemäßen Vorrichtung, wobei während der biochemischen Reaktion die Versorgungsflüssigkeit in der Versorgungskammer nicht einem äußerlich auferlegtem Druck unterliegt, so daß der molekulare Austausch zwischen der Versorgungskammer und den Lösungen der einzelnen Vertiefungen des inneren Gehäuses im wesentlichen auf Diffusion basiert.

Das erfindungsgemäße Verfahren bzw. die hierfür geeignete Vorrichtung eignet sich insbesondere für automatisierte Anwendungen mit hohem Synthesedurchsatz.

### Beispiel

Das erfindungsgemäße Verfahren zur zellfreien Proteinbiosynthese wird mit einem E. coli Lysat an zwei Modellproteinen (CAT und GFP) in folgendem näher erläutert:

Die zellfreie Proteinbiosynthese wird in Form einer gekoppelten Transkription und Translation durchgeführt, bei der die zu transkribierende mRNA auf einem Plasmid kodiert ist, dessen Gensequenz einen Promotor für eine virale RNA-Polymerase (z. B. SP6-, T3- oder T7-RNA-Polymerase) enthält.

Die in vitro transkribierte mRNA wird mit Hilfe des im gekoppelten Systems befindlichen E. coli Lysats in das entsprechende Protein translatiert.

### A) Reaktionskomponenten:

- *Plasmide:*: pM-GFP oder pIVEX-GFP enthalten die Sequenz für das Green Flourescent Protein aus Aequorea victoria in Form einer Mutante GFPcycle3 (27 kiloDalton) (Nature Biotechnology, 1996, 14, p.315-319); die kodierende Region der GFPcycle3 Mutante wurde in pTU58 anstelle der Wild-Typ GFP-Sequenz kloniert (Science, 1994, 263,802).
pHM-CAT enthält die Sequenz für das Chloramphenicol-Acetyltransferase-Protein (22,5 kiloDalton).
Konstruktion: Ein Insert (NcoI - BamHI) aus pCAT3 (Promega) wurde in pHM19 insertiert (FU Berlin, Insitut f. Biochemie, Dr. Stiege).
- *E.coli S30 Lysat*:: Das Lysat wurde mit einem E. coli A19-Stamm nach einem modifizierten Verfahren nach Zubay (Annu. Rev. Genet. 7, 267, 1973) präpariert.
Lysatpuffer: 100 mM Hepes-KOH pH 7,6/30°C, 14 mM Magnesium-acetat, 60 mM Kalium-acetat, 0,5 mM Dithiothreitol

### Zusammensetzung der Reaktions- und Versorgungslösung:

### Transkriptions-/Translations-Reaktionsansatz:

185 mM Kalium-acetat, 15mM Magnesium-acetat, 4% Glyzerin, 2,06 mM ATP, 1,02 mM CTP, 1,64 mM GTP, 1,02 mM UTP, 257 µM pro Aminosäure (insgesamt 20), 10,8 µg/ml Folinsäure, 1,03 mM EDTA, 100 mM Hepes-KOH pH 7,6/30°C, 1 µg/ml Rifampicin, 0,03% Natrium-azit, 40 mM Acetylphosphat, 480 µg/ml t RNA aus E.coli MRE600, 2mM Dithiothreitol, 10mM MESNA (Mercaptoethanesulfonic acid), 70 mM Kaliumhydroxid, 0,1 u/µl RNaseInhibitor, 15 µg/ml Plasmid, 220 µl/ml E.coli A19-Lysat, 2 u/µl T7 RNA Polymerase.

### Versorgungslösung:

185 mM Kalium-acetat, 15 mM Magnesium-acetat, 4% Glyzerin, 2,06 mM ATP, 1,02 mM CTP, 1,64 mM GTP, 1,02 mM UTP, 257 µM pro Aminosäure (insgesamt 20), 10,8 µg/ml Folinsäure, 1,03 mM EDTA, 100 mM Hepes-KOH pH 7,6/30°C,1 µg/ml Rifampicin, 0,03% Natrium-azit, 40 mM Acetylphosphat, 2 mM Dithiothreitol, 10 mM MESNA (Mercaptoethanesulfonic acid), 70 mM Kaliumhydroxid, Lysatpuffer wie oben beschrieben mit 220 µm/ml.

### B) Vorrichtung:

In dem hier beschriebenem Beispiel wurde ein Multi-channel-Dialyzer in Form einer Mikrotiterplatte eingesetzt. Die Böden der einzelnen Vertiefungen der Mikrotiterplatte (Wells) wurden mit einer Dialysemembran mit einem cut off-Volumen von 10 Kilo Dalton ausgestattet. Das maximale Reaktionsvolumen pro Well betrug 200 µl. Das Gehäuse für die Versorgungslösung hatte eine Volumenkapazität von 200 ml.

### C) Bewegung, Abdeckung:

Schüttelgerät: Flow Laboratories, Typ: Titertek® , mit der Schüttelfrequenz Stufe 4,5

Inkubationshaube: Edmund Bühler, Typ: TH25, mit der Temperatur 30°C

### D) Durchführung:

Es wurden getrennte Reaktionsansätze gemäß oben angegebener Zusammensetzung pipettiert:
1) Für die Expression von GFP mit pM-GFP bzw. mit pIVEX-GFP
2) Für die Expression von CAT mit pHM-CAT

Für jeden Ansatz wurde ein Volumen von 8,4 ml angesetzt und davon jeweils 200 µl in insgesamt 40 Wells pipettiert (0,4 ml als Überdosierung), das heißt 40 Wells pro Ansatz und Protein.

Für die Versorgungskammer wurden 200 ml Versorgungslösung gemäß oben angegebener Zusammensetzung pipettiert. Das heißt, es wurde eine gemeinsame Versorgungslösung für beide Reaktionsansätze verwendet.

Nach Abdichten der Mikrotiterplatte mit einer Klebefolie wurde die Vorrichtung auf das Schüttelgerät fixiert, welches sich unter der Inkubationshaube befand.

Nach Einstellen der Schüttelfrequenz und der Inkubationstemperatur von 30°C erfolgte eine 20 stündige Inkubation der Reaktionsansätze.

### E) Auswertung:

### 1) Expression von GFP:

Die Messung der Proben erfolgte mit einem Spektral-Fluorimeter der Fa. Kontron, Typ: TEGIMENTA, SFM25.

Die Anregung (Excitation) erfolgt bei einer Wellenlänge von 395 nm.

Die Emissionsrate bei 580 bis 430 nm.

Das Emissionsmaximum liegt bei 510 nm.

Als Standard wurde das rGFP (rekombinantes GFP) von Roche Diagnostics, Katalog-Nummer 1814524 verwendet.

Die Proben wurden mit dem Lysatpuffer 1 : 200, der Standard auf 1 µg/ml und 2 µg/ml verdünnt.

### Tabelle 1:

10 Werte der erhaltenen 40 (die alle im selben Bereich liegen) sind als representative Auswahl gezeigt.

### Abbildung 1:

Graphische Auswertung der in Tabelle 1 enthalenen Werte in µg GFP/ml Reaktionslösung in den exemplarisch ausgewählten Wells.

**Tabelle 1:**

| Well-Nummer | 1 | 2 | 3 | 4 | 5 | 6 | 7 | 8 | 9 | 10 |
|---|---|---|---|---|---|---|---|---|---|---|
| µg GFP/ml Reakt.vol. | 366 | 365 | 362 | 356 | 369 | 371 | 365 | 367 | 361 | 365 |

### 2) Expression von CAT:

Die Messung der Proben erfolgte über HPLC-Analyse mit dem Geräte-Typ:.LKB 2150.

Als Standard wurde ein CAT-Enzym von Roche Diagnostics, Katalog-Nummer 1485156 verwendet.

### Tabelle 2:

10 Werte der erhaltenen 40 (die alle im selben Bereich liegen) sind als representative Auswahl gezeigt.

### Abbildung 2:

Graphische Auswertung der in Tabelle 2 enthaltenen Werte in µg CAT/ml Reaktionslösung in den exemplarisch ausgewählten Wells.

**Tabelle 2:**

| Well-Nummer | 1 | 2 | 3 | 4 | 5 | 6 | 7 | 8 | 9 | 10 |
|---|---|---|---|---|---|---|---|---|---|---|
| µg CAT/ml Reaktionslsg. | 233 | 230 | 233 | 235 | 228 | 231 | 227 | 228 | 229 | 232 |

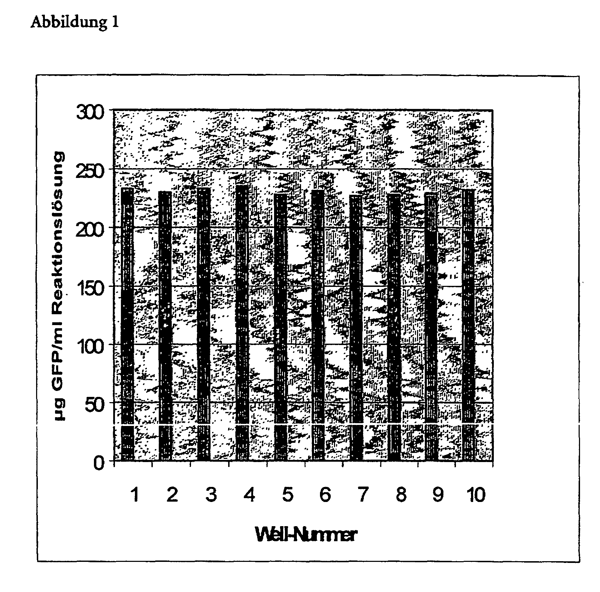

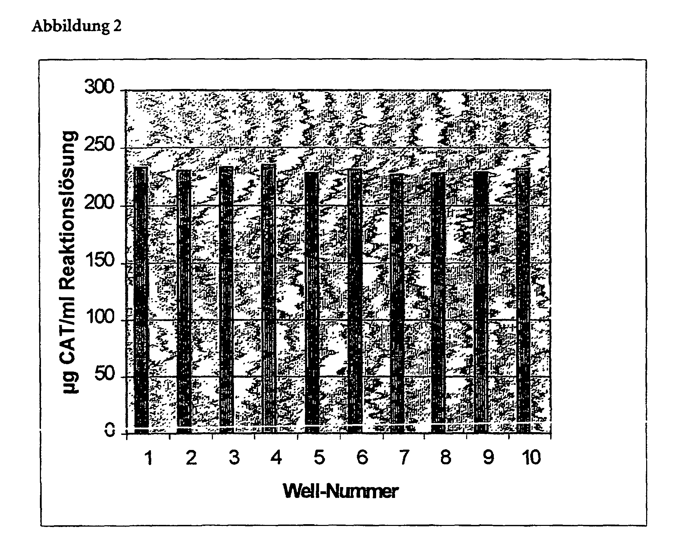

## Patentansprüche

1. Vorrichtung zur Durchführung zellfreier Polypeptid-Biosynthesen, bestehend aus einem äußeren Gehäuse, das ein inneres Gehäuse mit eingearbeiteten Vertiefungen und eine Versorgungskammer umschließt, wobei die Vertiefungen des inneren Gehäuses während der biochemischen Reaktion jeweils ein produzierendes System enthalten, die Versorgungskammer während der biochemischen Reaktion eine Versorgungsflüssigkeit enthält und die Vertiefungen des inneren Gehäuses und die Versorgungskammer durch eine semipermeable Membran getrennt sind, **dadurch gekennzeichnet, daß** das innere Gehäuse mindestens zwei Vertiefungen aufweist, deren untere Enden mit der semipermeablen Membran abgeschlossen sind und deren obere Enden aus der in der Versorgungskammer enthaltenen Versorgungsflüssigkeit herausragen, und mit Mitteln zur Bewegung und Temperierung der produzierenden Systeme sowie der Versorgungsflüssigkeit verbunden ist, wobei die Mittel zur Bewegung dergestalt sind, dass die Durchmischung des produzierenden Systems und der Versorgungslösung simultan erfolgt.

2. Vorrichtung nach Anspruch 1, **dadurch gekennzeichnet, daß** das innere Gehäuse zwischen zwei und 1000 Vertiefungen aufweist.

3. Vorrichtung nach Anspruch 1 oder 2, **dadurch gekennzeichnet, daß** die Vertiefungen an den Seitenwänden mit einer die in vitro synthetisierten Proteine spezifisch bindenden Komponente beschichtet sind.

4. Vorrichtung nach Anspruch 3, **dadurch gekennzeichnet, daß** die Vertiefungen mit solchen Komponenten beschichtet sind, die zur Reinigung von tag-enthaltenen Proteinen geeignet sind.

5. Vorrichtung nach Anspruch 3 oder 4, **dadurch gekennzeichnet, daß** die Vertiefungen mit Streptactin, Avidin oder Streptavidin beschichtet sind.

6. Vorrichtung nach einem der Ansprüche 1-5, **dadurch gekennzeichnet, daß** die Vertiefungen des inneren Gehäuses jeweils ein Volumen zwischen 50 µl und 10 ml aufweisen.

7. Vorrichtung nach einem der Ansprüche 1-6, **dadurch gekennzeichnet, daß** das Volumen der Versorgungslösung das fünf- bis zwanzig-fache der Summe der Volumina der Vertiefungen entspricht.

8. Vorrichtung nach einem der Ansprüche 1-7, **dadurch gekennzeichnet, daß** es sich bei der semipermeablen Membran um eine Dialysemembran oder Ultrafiltrationsmembran mit einer Porengröße von 3 bis 100 kDa handelt.

9. Vorrichtung nach einem der Ansprüche 1-8, **dadurch gekennzeichnet, daß** zur Abdichtung der Öffnungen der Vertiefungen des inneren Gehäuses jede Vertiefung mit einer Verschlußkappe oder Folie versehen ist oder ein Verschlußdeckel zur Abdichtung des äußeren Gehäuses vorhanden ist.

10. Vorrichtung nach Anspruch 1, **dadurch gekennzeichnet, daß** die Durchmischung durch ein Schüttel- oder Rührelement erfolgt.

11. Verfahren zur Durchführung einer oder mehrerer zellfreier Polypeptid-Biosynthesen parallel mittels einer Vorrichtung nach einem der Ansprüche 1-10, **dadurch gekennzeichnet, daß** während der zellfreien Polypeptid-Biosynthese die Versorgungsflüssigkeit in der Versorgungskammer nicht einem äußerlich auferlegtem Druck unterliegt, so daß der molekulare Austausch zwischen der Versorgungskammer und den einzelnen Vertiefungen des inneren Gehäuses im wesentlichen auf Diffusion basiert.

12. Verfahren nach Anspruch 11 in Verbindung mit Anspruch 10, **dadurch gekennzeichnet, daß** die Versorgungsflüssigkeit und gegebenenfalls das produzierende System in jeder der vorhandenen Vertiefungen des inneren Gehäuses während der zellfreie Polypeptid-Biosynthese mittels eines Magnetrührelement bewegt wird.

## Claims

1. Device for carrying out cell-free polypeptide biosyntheses composed of an external housing which encloses an inner housing with incorporated wells and a supply chamber, wherein the wells of the inner housing each contain a producing system during the biochemical reaction, the supply chamber contains a supply liquid during the biochemical reaction and the wells of the inner housing and the supply chamber are separated by a semipermeable membrane, **characterized in that** the inner housing has at least two wells the lower ends of which are closed by a semipermeable membrane and the upper ends of which protrude from the supply liquid contained in the supply chamber, and is connected to means for moving and incubating the producing systems and the supply liquid, said means for moving being such that the producing system and supply solution are mixed simultaneously.

2. Device as claimed in claim 1, **characterized in that** the inner housing has between 2 and 1000 wells.

3. Device as claimed in claim 1 or 2, **characterized in that** the side walls of the wells are coated with a component which specifically binds the in vitro synthesized proteins.

4. Device as claimed in claim 3, **characterized in that** the wells are coated with components that are suitable for purifying tag-containing proteins.

5. Device as claimed in claim 3 or 4, **characterized in that** the wells are coated with streptactin, avidin or streptavidin.

6. Device as claimed in one of the claims 1-5, **characterized in that** the wells of the inner housing each have a volume between 50 µl and 10 ml.

7. Device as claimed in one of the claims 1-6, **characterized in that** the volume of the supply solution is five to twenty-times the sum of the volumes of the wells.

8. Device as claimed in one of the claims 1-7, **characterized in that** the semipermeable membrane is a dialysis membrane or an ultrafiltration membrane with a pore size of 3 to 100 kDa.

9. Device as claimed in one of the claims 1-8, **characterized in that** in order to seal the openings of the wells of the inner housing, each well is provided with a cap closure or foil or a closing cover is present to seal the outer housing.

10. Device as claimed in claim 1, **characterized in that** the mixing is achieved by a shaking or stirring element.

11. Method for carrying out one or more cell-free polypeptide biosyntheses concurrently by means of a device as claimed in one of the claims 1-10, **characterized in that** the supply liquid in the supply chamber is not subjected to an external applied pressure during the cell-free polypeptide biosynthesis and thus the molecular exchange between the supply chamber and the individual wells of the inner housing is essentially based on diffusion.

12. Method as claimed in claim 11 in conjunction with claim 10, **characterized in that** the supply liquid and optionally the producing system in each of the wells of the inner housing are moved during the cell-free polypeptide biosynthesis by means of a magnetic stirring element.

## Revendications

1. Dispositif pour la mise en oeuvre de biosynthèses de polypeptides acellulaires, constitué par un boîtier extérieur qui renferme un boîtier intérieur à puits incorporés et un compartiment d'alimentation, dispositif dans lequel les puits du boîtier intérieur contiennent chacun, pendant la réaction biochimique, un système de production, le compartiment d'alimentation contient, pendant la réaction biochimique un liquide d'alimentation, et les puits du boîtier intérieur sont séparés du compartiment d'alimentation par une membrane semi-perméable, **caractérisé en ce que** le boîtier intérieur présente au moins deux puits dont les extrémités inférieures sont fermées par la membrane semi-perméable et dont les extrémités supérieures dépassent le niveau du liquide d'alimentation contenu dans le compartiment d'alimentation, et qu'il est relié à des moyens d'agitation et de mise à température des systèmes de production ainsi que du liquide d'alimentation, e n ce que le brassage du système de production et de la solution d'alimentation sont réalisés simultanément.

2. Dispositif selon la revendication 1, **caractérisé en ce que** le boîtier intérieur présente de 2 à 1000 puits.

3. Dispositif selon la revendication 1 ou 2, **caractérisé en ce que** les parois latérales des puits sont revêtues d'un composant qui se lie d'une manière spécifique à l'une des protéines synthétisées in vitro.

4. Dispositif selon la revendication 3, **caractérisé en ce que** les puits sont revêtus de composants qui sont appropriés pour la purification de protéines contenant des marqueurs.

5. Dispositif selon la revendication 3 ou 4, **caractérisé en ce que** les puits sont revêtus de streptactine, d'avidine ou de streptavidine.

6. Dispositif selon l'une quelconque des revendications 1 à 5, **caractérisé en ce que** les puits du boîtier intérieur présentent chacun un volume compris entre 50 µl et 10 ml.

7. Dispositif selon l'une quelconque des revendications 1 à 6, **caractérisé en ce que** la solution d'alimentation présente un volume de cinq à vingt fois supérieur à la somme des volumes des puits.

8. Dispositif selon l'une quelconque des revendications 1 à 7, **caractérisé en ce que** la membrane semi-perméable est une membrane de dialyse ou une membrane d'ultrafiltration ayant une taille de pores de 3 à 100 kDa.

9. Dispositif selon l'une quelconque des revendications 1 à 8, **caractérisé en ce que** pour le scellement des ouvertures des puits du boîtier intérieur, chaque puits est muni d'un capuchon ou d'un film ou bien il existe un couvercle pour le scellement du boîtier extérieur.

10. Dispositif selon la revendication 1, **caractérisé en ce que** le brassage est réalisé à l'aide d'une secoueuse ou d'un agitateur.

11. Procédé pour la mise en oeuvre en parallèle d'une ou plusieurs biosynthèses de polypeptides acellulaires, à l'aide d'un dispositif selon l'une quelconque des revendications 1 à 10, **caractérisé en ce que** pendant la biosynthèse de polypeptides acellulaires, le liquide d'alimentation dans le compartiment d'alimentation n'est p as soumis à une pression exercée de l'extérieur, de sorte que l'échange moléculaire entre le compartiment d'alimentation et les puits individuels du boîtier intérieur est essentiellement basé sur la diffusion.

12. Procédé selon la revendication 11 en liaison avec la revendication 10, **caractérisé en ce que** le liquide d'alimentation et le cas échéant, le système de production dans chacun des puits présents dans le boîtier intérieur sont agités au moyen d'un agitateur magnétique pendant la biosynthèse de polypeptides acellulaires.
